# EUROPEAN PATENT APPLICATION

(11) **EP 1 421 961 A2**
(43) Date of publication of application: **26.05.2004**
(21) Application number: 03025865.1
(22) Date of filing: 11.11.2003
(51) Int. Cl.: A61M 5/178, A61J 1/20

(54) **Self-aligning shield for syringe**

(30) Priority: 14.11.2002 US 294978
(71) Applicant: Becton, Dickinson and Company, Franklin Lakes, New Jersey 07417-1880 (US)
(72) Inventor: DiBiasi, Michael A., West Milford New Jersey 07480 (US); Phan, Vu, Edison New Jersey 08817 (US); Scovell, Daniel B., West Grove Pennsylvania 19390 (US)
(74) Representative: von Kreisler, Alek, Dipl.-Chem.

(57) **Abstract**

The present invention is a shield that protects the needle of a syringe and maintains it in a sterile condition until use. The shield is able to become open at both ends. A paper or thermoplastic barrier maintains sterility and keeps the distal end of the shield closed prior to use. During usage of the present invention, the distal end of the shield also serves as a guide over the neck of a vial. The present invention is a shield for a syringe having a shield, which has two sterility barriers, where one of the sterility barriers is removable from the shield, and the other is formed at an interface between a syringe and the shield. In addition, the shield also has a guidance section that is adapted to guide a vial crimp into the shield for interface to a needle.

## Description

### Field of the Invention

The present invention relates to syringes and more particularly to shields for syringes, further in particular, syringes with short needles.

### Background of the Invention

Generally speaking, a hypodermic syringe consists of a cylindrical barrel, most commonly made of thermoplastic material or glass, having a distal end connected to a sharp needle cannula or adapted to be connected to a hypodermic needle assembly and a proximal end adapted to receive a resilient stopper and plunger rod assembly. Fitting over the distal end of the syringe for protection of sterility is a needle shield or shield. The shield is used to both protect the user from needle sticks as well as provide for a sterile barrier in self-contained syringes. Self-contained syringes are a special variety of syringes such that an integral sterility barrier is designed into the proximal (plunger) and distal ends (needle). In order to deliver a dosage of medicament, one penetrates a vial containing medicament through a vial stopper and draws a dosage of medicament from the interior of the vial into the syringe. Vial stoppers are designed generally of an elastomeric material, and may have a variable cross section. It is preferred to penetrate the vial at the center of the vial stopper.

Increasingly needles are becoming shorter and shorter. Needles as short as 5mm are being used to deliver insulin (See US Patent 6,200,291 DiBiasi et. al.) The '291 patent describes a reusable pen syringe needle of 5mm length used to deliver insulin. Some medicaments are only available in vials, and if one wanted to deliver a medicament at a 5mm depth one would need to use a 5mm usable length syringe, fillable via the distal end. However, there is a problem, if one were to utilize a 5mm needle on a disposable syringe, which needs to be filled via the distal end, one would find that the needle just barely penetrates the stopper of a standard insulin vial which can be slightly less than 5mm in thickness. Furthermore, if one were to penetrate the vial at an angle one could conceivably not fully penetrate the vial stopper and not gain fluid communication to the contents of the vial in order to draw the dose into the syringe. Furthermore, if one were to penetrate the vial off-center one could conceivably not fully penetrate the vial stopper, since vial stoppers are of varying cross sectional thicknesses, and generally are thinnest at the center. What is needed is a device to align the syringe with the vial such that a needle is penetrated substantially perpendicular to the vial stopper and at the center of the stopper so that the needle depth into the vial interior may be maximized.

Vial adapters to aid in penetration of vials have been proposed in the past. Various designs have been proposed in the past to align the vial to the syringe. One example of such a device is related in US Patent 5,356,406 Shraga. The design of this adapter is such that it provides guidance of the needle to the vial. The vial adapter in the '406 patent is not integral to the syringe, which may facilitate in its loss. In addition, the vial adapter of '406 does not maintain the sterility of the needle or shield the needle prior to usage, since it is not sterilized with the syringe. Another such example of a vial adapter is related in US Patent 4,944,736 Holtz. The design of this adapter is such that it provides guidance of the needle to the vial. The vial adapter in the '736 patent is not integral to the syringe, which may facilitate in its loss. Although the vial adapter of the '736 patent relates a removable sterility barrier, it does not maintain sterility of the vial since it is not sterilized with the vial. In addition, the vial adapter of '736 does not maintain the sterility of the needle or shield the needle prior to usage, as it is a separate component, which is not sterilized with the syringe. What is needed is a device that both integrates syringe sterility assurance with guidance means to the vial.

### Summary of the Invention

The present invention is a device that protects the needle of a syringe and maintains it in a sterile condition until use. Unlike a standard syringe shield, which is closed at one end, the self-aligning shield of the present invention is able to become open at both ends. A paper or thermoplastic barrier maintains sterility and keeps the distal end of the shield closed prior to use. The distal end also serves as a guide over the neck of a vial. One object of the present invention is to ensure that the needle penetrates substantially perpendicular to the vial stopper, as well as in the center of the vial stopper. Another object of the present invention is to provide a safety feature, which makes proper technique compulsory. Forcing proper technique assures patients and nurses that the needle will always breach the vial stopper and allow the proper dosing of medicament from the interior of the vial to the syringe. Yet another object of the invention is to provide support to the vial itself during aspiration of the syringe. The user is not required to support the vial with a free hand during syringe aspiration, and since the vial is engaged to the syringe in a more stable fashion, therefore, only one hand may be used for filling. In addition, one may more readily extract a greater portion of the medicament from the vial since the syringe needle can be accurately placed at the lowest portion of the vial, while using the present invention.

Yet another object of the invention is to maintain sterility of the needle in the same fashion that a self-contained shield maintains sterility of the needle. The present invention maintains this sterility by the use of a removable sterility barrier. The removable sterility barrier maintains the sterility of the needle as well as provides for access to the needle by the vial for filling of the syringe.

The present invention offers significant advantages over the prior art in terms of safety and convenience. With conventionally shielded syringes, there is a practical limit on the length of the cannula. For some patients, a shorter cannula offers better injection comfort and eliminates the need for pinching up the tissue at the injection site. However, with shorter cannula, there is an increased risk of user error. Improper technique could include inserting the needle off center or at an angle, which increases the likelihood that the short needle will not breach the vial stopper. It is also important to note that the same needle that pierces the vial stopper is the same needle that enters the patient.

The present invention is not removed prior to drawing the medicament from the vial, as with a conventional shield; rather, it stays on the syringe until after the dose is drawn. While still attached to the syringe, the present invention ensures that even the short variety of cannula will breach the vial stopper. The dimensions of the vial interfacing end of the shield aid in targeting the vial's neck. For example, instead of attempting to target a 5mm diameter target at the vial stopper center with a .4mm diameter needle, one merely has to target a 15mm diameter target of the present invention with a 14mm vial crimp.

The present invention also supports the vial such that it does not need to be supported by the free hand during the drawing of medicament. By allowing the patient to ignore the vial balancing act and concentrate on drawing an accurate dose, possibilities for error are reduced. In addition, the wide end of the shield allows it to be very stable when at rest on a flat surface. This makes it easier to perform a one-handed re-shield, although the practice of re-shielding is not recommended.

The above described disadvantages are overcome, and other advantages are realized, in a system and method according to the present invention.

### Brief Description of the Drawings

These and other features, aspects, and advantages of the present invention will become better understood with regard to the following description, appended claims, and accompanying drawings where:
Fig. 1, illustrates a side view of a shield of the present invention
Fig. 2 illustrates a cross-sectional view of the shield of Fig 1.
Fig. 3 illustrates the shield of Fig 1 attached to a syringe in an isometric view.
Fig. 4 illustrates an alternate view of the distal end of the shield and syringe assembly of Fig 3.
Fig. 5 illustrates a side view of a shield of the present invention assembled to a syringe in use.
Fig 6. illustrates the assembly of Fig 5. in use, attached to a vial
Fig 7. illustrates a partial cross-sectional view of the shield, syringe and vial assembly of Fig. 6. noted as detail A-A in Fig. 6.

In the drawing figures, it will be understood that like numerals refer to like features and structures.

### Detailed Description of the Preferred Embodiments

Preferred embodiments of the present invention will now be described with reference to the attached drawing figures.

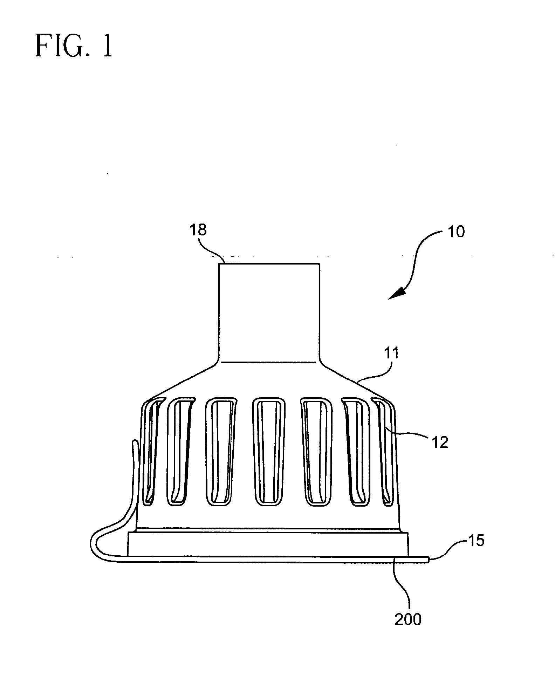

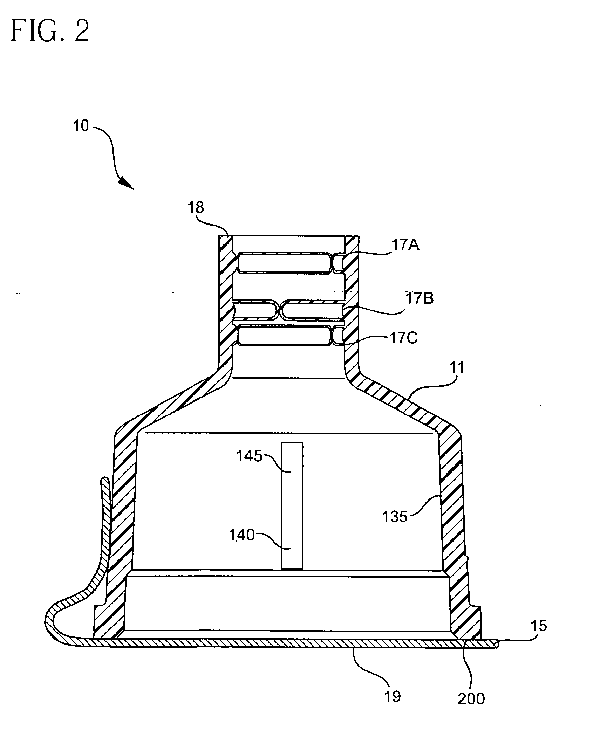

As shown in Figs. 1 and 2, a self aligning shield 10, herein referred to as shield 10 is comprised of two elements: body 11 and label 15. Typically, label 15 is hermetically sealed to body 11 at distal end 19 of body 11 via a heat and pressure technique. Body 11 is typically molded of a thermoplastic polymer, while label 15 is typically a laminated structure designed to facilitate a heat seal to plastic materials. Alternatively, a non-laminated material for label 15 may be utilized such as a solid plastic component or a single layer polymeric sheet. Body 11 and label 15 are assembled to form first sterility barrier 200, which is at the interface of label 15, and body 11. It is always possible to remove first sterility barrier 200 by the patient in order to utilize the guidance aspect of the present invention.

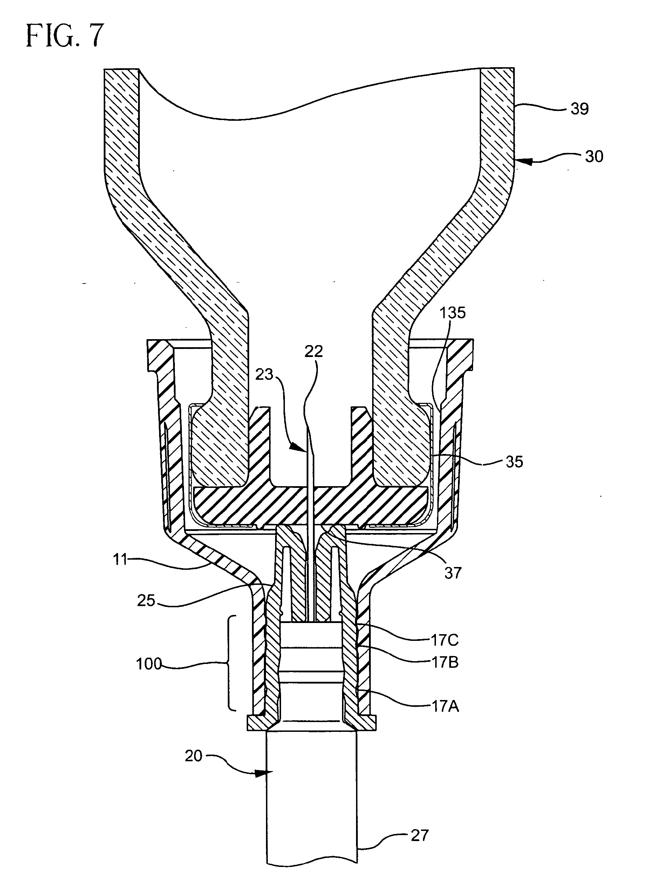

Also shown are optional ribs 12 integrally molded into body 11; ribs 12 facilitate easy handling of shield 10. Proximal end 18 of body 11 is typically assembled on to a barrel or hub of a syringe during manufacture of the syringe. The syringe and shield 10 assembly is then sterilized. At proximal end 18 of shield 10, is at least one toroidal ring 17. Shown in the figure are a plurality of toroidal rings, 17A, 17B, and 17C which facilitate a labyrinth path, also known as a tortuous path, a type of sterility barrier which is well know in the art. Rings 17 may substantially encircle a portion of a syringe to which shield 10 is assembled. Alternatively, in the case of a single ring 17, a hermetic seal between shield 10 and the syringe to which it is assembled would be formed. Ring(s) 17 along with features on the syringe form a second sterility barrier 100, which is formed at the interface between shield 10 and the syringe to which shield 10 is assembled. Second sterility barrier 100 is shown more clearly in Fig. 7. Since an object of the present invention is to provide a sterility barrier for the needle end of a syringe, both first sterility barrier 200 and second sterility barrier 100 ensure the sterility of the needle, since there are no other paths for the microbes to enter the interior portion of shield 10.

Another feature disposed in body 11 is crimp surface 135, which is sized to be slightly larger than a standard vial crimp 35. Crimp surface 135 is a cylindrical section of body 11 which is approximately the length and diameter of a vial crimp to which shield 10 will be interfaced. Optionally, crimp surface 135 may also be tapered to be larger in diameter at distal end 19 of body 11 and tapering to a diameter slightly larger than crimp 35 at proximal end of crimp surface 135 to aid in guidance of crimp 35 into body 11. In an alternate embodiment, crimp surface 135 is non-continuous and is formed from portions of a plurality of crush rib(s) 140 by a diameter of crush rib contact surface(s) 145. Non-continuous crush rib surface(s) 140 form a diameter, which interfaces with crimp 35, at the same critical diameter that a continuous crimp surface 135 interfaces with crimp 35 in the previous embodiment.

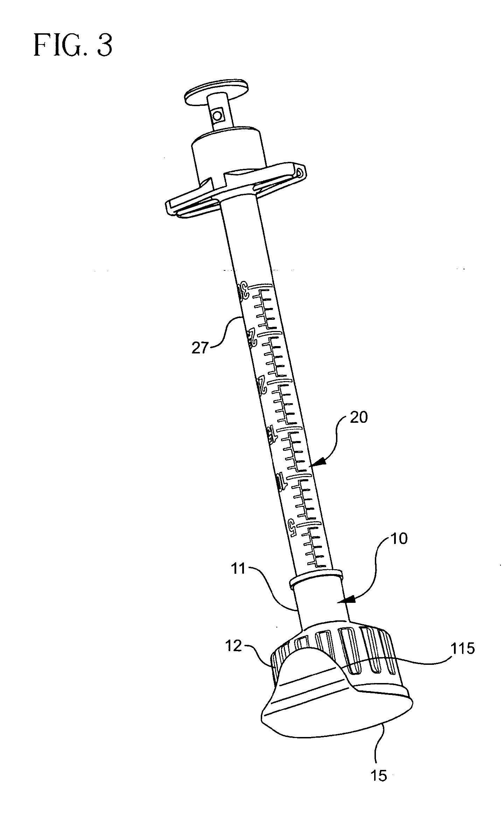

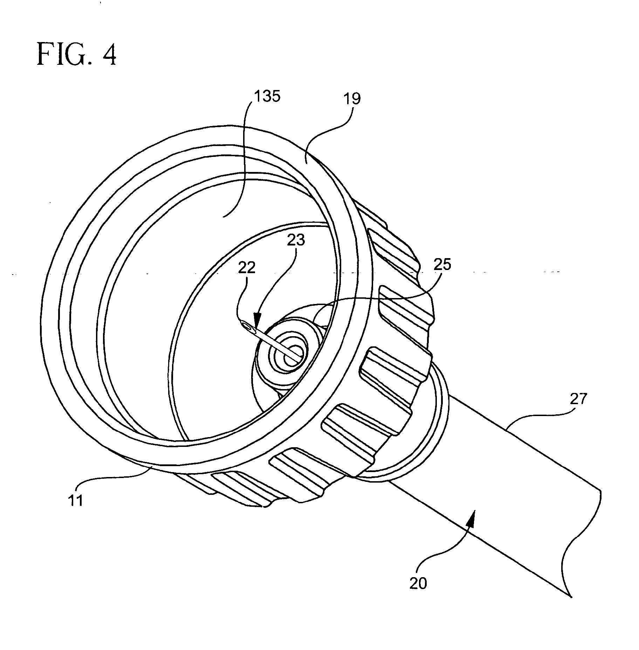

Shown in Fig. 3 is a syringe 20 to which a shield 10 of the present invention has been assembled. Label 15 is attached to body 11 at distal end 19 and a small portion of label 15 called tab 115, is used as a pull tab by the patient's fingers. Tab 115 is optionally attached to rib 12 with a heat stake to prevent unintentional breaching of sterility barrier 200. During usage, the patient grasps ribs 12 and pulls tab 115 to remove label 15 from body 11. This intermediate condition with label 15 removed from body 11 is shown in Fig. 4. Removal of label 15 from body 11 exposes needle 23 and distal tip 22 of needle 23, which allow access to a vial. Also shown in Fig 4 is hub 25 which is a feature disposed on syringe 20 which serves two primary functions: to hold needle 23 as well as to provide a cylindrical surface to interface to rings 17 to provide for second sterility barrier 100.

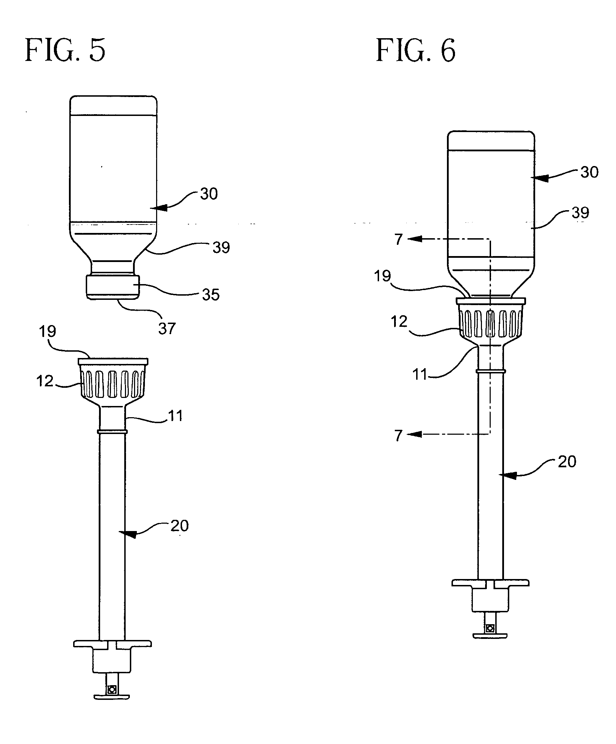

Fig. 5 shows an intermediate step of the usage of the present invention, while Figs. 6 & 7 show the completed interface between vial 30 and syringe 20 (to which shield 10 has been assembled). Referring to these figures, which show a typical vial 30 which is comprised of crimp 35 which holds vial stopper 37 onto glass 39. Alternatively, vial 30 could be any reservoir of liquid to be filled into a syringe with an access point similar in size and configuration to that of a standard vial. Vial 30 is positioned such that vial stopper 37 at the proximal end of vial 30 is positioned to be penetrated by distal tip 22 of needle 23. The patent holds body 11 while moving vial 30 axially in the proximal direction towards distal end 19 of body 11. Since crimp surface 135 is sized to be slightly larger in inner diameter than the outer diameter of crimp 35, crimp 35 is allowed to enter body 11 of shield 10. This is critical to the invention in that this is how shield 10 provides guidance of vial 30 onto syringe 20. Since there is little clearance between the diameters of crimp 35 and crimp surface 135, a substantially perpendicular penetration by needle 23 of vial stopper 37 is assured. Continuing axially, vial stopper 37 is penetrated by distal tip 22 of needle 23. At the end of the penetration of vial stopper 37 by needle 23, as shown in Fig. 6 and 7, distal tip 22 of needle 23 is past the inner surface of vial stopper 37 and allows fluid communication between the contents of vial 30 and syringe 20. Also shown in Fig. 7 is second sterility barrier 100, which is formed in the interface of rings 17 and hub 25. As shown in the figure, hub 25 is a separate component to barrel 27 to form syringe 20, although they could be a single integral barrel on syringe 20.

The invention has now been described in detail, however, it will be appreciated that certain changes and modifications may be made. For example, although illustrated in the context of enclosing the shield with a paper label, other methods of maintaining a removable sterility barrier at the vial interfacing end may be employed, such as designing an integrated, hinged element made from the shield material itself.

While the invention herein disclosed has been described by means of specific embodiments and applications thereof, numerous modifications and variations could be made thereto by those skilled in the art without departing from the scope of the invention set forth in the claims.

## Claims

1. A shield for a syringe comprising:
a. a body having a distal end and a proximal end;
b. said body having a first sterility barrier at said distal end wherein said sterility barrier is removable from said body;
c. said body having a second sterility barrier at said proximal end wherein said second sterility barrier is formed at an interface between said syringe and said body;
d. said body having a guidance section wherein said guidance section is adapted to guide a reservoir from said distal end towards said proximal end.

2. The shield according to claim 1 wherein said shield is removably attached to said syringe.

3. The shield according to claim 1 wherein said guidance section is tapered.

4. The shield according to claim 1 wherein said first sterility barrier is composed of a laminate material.

5. The shield according to claim 4 wherein said laminate material is hermetically sealed to said body.

6. The shield according to claim 1 wherein said first sterility barrier is a polymeric material.

7. The shield according to claim 1 wherein said second sterility barrier is a plurality of rings forming a tortuous path.

8. The shield according to claim 1 wherein said second sterility barrier is at least one ring forming a hermetic seal to said syringe.

9. A shield for a syringe comprising:
a. a body having a distal end and a proximal end;
b. said body having a first sterility barrier composed of a laminate of paper and polymer wherein said sterility barrier is hermetically sealed to said body at said distal end and said first sterility barrier is removable from said body;
c. said body having a second sterility barrier wherein said second sterility barrier is at said proximal end and is composed of a plurality of rings formed on said body at an interface between said syringe and said body to form a tortuous path;
d. said body having a guidance section wherein said guidance section tapered and said adapted to guide a reservoir into said body for interface to a needle and
e. wherein said shield is removably attached to said syringe.

10. A method of filling a syringe using a shield comprising:
a. removing a first sterility barrier on said shield;
b. accessing a reservoir with said syringe using said shield as a guide;
c. filling said syringe attached to said shield;
d. and removing said shield from said syringe.
